# EUROPEAN PATENT APPLICATION

(11) **EP 4 659 667 A1**
(43) Date of publication of application: **10.12.2025**
(21) Application number: 24770653.4
(22) Date of filing: 05.03.2024
(51) Int. Cl.: A61B 5/11, A61B 5/113, G01V 3/12

(54) **PROCESSING APPARATUS, PROCESSING METHOD, AND PROGRAM**

(30) Priority: 13.03.2023 JP 2023038763
(71) Applicant: OMRON Corporation, Kyoto 600-8530 (JP)
(72) Inventor: MATSUURA, Keiki, Kyoto-shi, Kyoto 600-8530 (JP); KAWAKAMI, Riho, Kyoto-shi, Kyoto 600-8530 (JP); IWADE, Ayaka, Kyoto-shi, Kyoto 600-8530 (JP); OZAWA, Hisashi, Kyoto-shi, Kyoto 600-8530 (JP)
(74) Representative: Isarpatent
(86) International application number: PCT/JP2024/008364
(87) International publication number: WO 2024/190543

(57) **Abstract**

To calculate time-series data of the vital index for each person. A processing apparatus includes: a signal acquisition unit that acquires a vital signal from a signal reflected by at least one person within a measurement range; an index computation unit that computes a vital index representing a vital state of the at least one person based on the vital signal; a body movement determination unit that determines the presence or absence of a body movement of the at least one person within the measurement range; a person count estimation unit that estimates the number of the at least one person within the measurement range; a person count change determination unit that determines the presence or absence of a possibility of a change in the estimated number of the at least one person when the presence is determined of the body movement of the at least one person within the measurement range; and a calculation unit that calculates time-series data of the vital index for each of the at least one person. When the presence is determined of the possibility of the change in the estimated number of the at least one person, the calculation unit separately calculates the time-series data of the vital index before determining the presence of the possibility of the change in the estimated number of the at least one person, and the time-series data of the vital index after determining the presence of the possibility of the change in the estimated number of the at least one person.

## Description

### TECHNICAL FIELD

The present invention relates to a processing apparatus, a processing method, and a program.

### BACKGROUND ART

For example, PTL 1 proposes a technique in which signal components are separated from phase signals obtained from radar signals, a target vital signal is identified based on temporal characteristics such as repetitiveness and frequency characteristics of the separated signal components, and a vital index is calculated.

### CITATION LIST

### PATENT LITERATURE

PATENT LITERATURE 1: International Publication Pamphlet No. WO 2021/171091

### SUMMARY OF INVENTION

### TECHNICAL PROBLEM

By acquiring vital indices in a time series manner, time-series data of the vital index is calculated. When the number of subjects to be measured is unknown or indeterminate, it is possible that vital signals and vital indices for a plurality of persons will be detected. When acquiring vital indices in a time series manner, it is possible that the number of acquired vital indices changes depending on the time. For example, the number of acquired vital indices at a first point in time may differ from the number at a second point in time. A possible cause of the change in the number of acquired vital indices is an actual change in the number of subjects. In known techniques, time-series data of vital index is not calculated in consideration of the possibility of a change in the number of subjects, and therefore it is difficult to calculate time-series data of vital index for each person. In view of the above circumstances, an object of the present invention is to provide a technique capable of calculating time-series data of vital index for each person.

### SOLUTION TO PROBLEM

A processing apparatus according to an aspect of the present invention includes: a signal acquisition unit configured to acquire a vital signal from a signal reflected by at least one person within a measurement range; an index computation unit configured to compute a vital index representing a vital state of the at least one person based on the vital signal; a body movement determination unit configured to determine the presence or absence of a body movement of the at least one person within the measurement range; a person count estimation unit configured to estimate the number of the at least one person within the measurement range; a person count change determination unit configured to determine the presence or absence of a possibility of a change in the estimated number of the at least one person when the presence is determined of the body movement of the person within the measurement range; and a calculation unit configured to calculate time-series data of the vital index for each of the at least one person. When the presence is determined of the possibility of the change in the estimated number of the at least one person, the calculation unit separately calculates the time-series data of the vital index before determining the presence of the possibility of the change in the estimated number of the at least one person, and the time-series data of the vital index after determining the presence of the possibility of the change in the estimated number of the at least one person.

According to the processing apparatus, in the case where the time-series data of the vital index is calculated for each person and there is a possibility of a change in the number of the at least one person within the measurement range, the time-series data of the vital index before determining the presence of the possibility of the change in the number of the at least one person and the time-series data of the vital index after determining the presence of the possibility of the change in the number of the at least one person are separately calculated. In this manner, the time-series data of the vital index corresponding to the number of the at least one person before the number of the at least one person has changed and the time-series data of the vital index corresponding to the number of the at least one person after the number of the at least one person has changed can be separately calculated for each person.

In the processing apparatus according to an aspect of the present invention, the body movement determination unit may compute a body movement time of the at least one person within the measurement range, and the person count change determination unit may determine the presence or absence of the possibility of the change in the estimated number of the at least one person based on the body movement time of the at least one person. For example, when the body movement time of the at least one person is short, there is no possibility of the change in the estimated number of the at least one person, whereas when the body movement time of the person is long, there is a possibility of the change in the estimated number of the at least one person. According to the processing apparatus, by determining the presence or absence of the possibility of the change in the estimated number of the at least one person based on the body movement time of the at least one person, the presence or absence of the possibility of the change in the estimated number of the at least one person can be simply determined.

In the processing apparatus according to an aspect of the present invention, the index computation unit may compute the vital index for each of a plurality of periods, the person count estimation unit may estimate the number of the at least one person within the measurement range for each of the plurality of periods, the body movement determination unit may determine the presence or absence of the body movement of the at least one person within the measurement range for each of the plurality of periods, and the calculation unit may calculate the time-series data of the vital index for each of the at least one person by excluding a period in which the vital index corresponding to the estimated number of the at least one person has not been computed from a period in which the absence is determined of the body movement of the at least one person within the measurement range among the plurality of periods. It is possible that the time-series data of the vital index for the period in which the vital index corresponding to the estimated number of the at least one person has not been computed is low-reliability data. According to the processing apparatus, by calculating the time-series data of the vital index for each of the at least one person by excluding the period in which the vital index corresponding to the estimated number of the at least one person has not been computed, the reliability of the time-series data of the vital index can be improved.

The processing apparatus according to an aspect of the present invention may further include a location information computation unit configured to identify a position of the at least one person within the measurement range, and compute location information of the at least one person associated with the vital index for each of the at least one person. The index computation unit may compute the vital index for each of a plurality of periods, the location information computation unit may compute the location information for each of the plurality of periods, and the calculation unit may determine a combination of the vital index for each of the at least one person by associating the vital index for each of the at least one person based on the location information for each of the plurality of periods, and may calculate the time-series data of the vital index for each of the at least one person based on the combination of the vital index. In this manner, the time-series data of the vital index can be calculated for each of the at least one person.

In the processing apparatus according to an aspect of the present invention, the index computation unit may compute the vital index for each of a plurality of periods, and the calculation unit may determine a combination of the vital index for each of the at least one person by associating the vital index for each of the at least one person based on a value of the vital index for each of the plurality of periods, and may calculate the time-series data of the vital index for each of the at least one person based on the combination of the vital index. In this manner, the time-series data of the vital index can be calculated for each of the at least one person.

The processing apparatus according to an aspect of the present invention may further include an index output unit configured to output the time-series data of the vital index for each of the at least one person. When the presence is determined of the possibility of the change in the estimated number of the at least one person, the index output unit may separately output the time-series data of the vital index before determining the presence of the possibility of the change in the estimated number of the at least one person, and the time-series data of the vital index after determining the presence of the possibility of the change in the estimated number of the at least one person. In this manner, the time-series data of the vital index corresponding to the number of the at least one person before the number of the at least one person has changed and the time-series data of the vital index corresponding to the number of the at least one person after the number of the at least one person has changed can be separately output for each of the at least one person.

In the processing apparatus according to an aspect of the present invention the signal acquisition unit may execute a process of clustering a vital waveform group into a plurality of clusters based on a feature computed from the vital signal, the vital waveform group being acquired based on the vital signal, and the person count estimation unit may estimate the number of the at least one person within the measurement range based on the number of clusters. In the processing apparatus according to an aspect of the present invention the body movement determination unit may determine the presence or absence of the body movement of the at least one person within the measurement range for each of the plurality of periods, and the person count estimation unit may estimate the number of the at least one person within the measurement range for each of the plurality of periods, and estimate that a maximum value of the number of clusters is the number of the at least one person within the measurement range for a period in which the absence is determined of the body movement of the at least one person within the measurement range among a continuous period in the plurality of period. In a period in which the absence is determined of the body movement of the at least one person within the measurement range among the continuous period in the plurality of periods, it is possible to estimate the absence of the change in the number of the at least one person, and therefore it is estimated that the maximum value of the number of clusters is the number of the at least one person within the measurement range.

The processing apparatus according to an aspect of the present invention may further include an abnormality detection unit configured to detect a vital abnormality of the at least one person within the measurement range based on the estimated number of the at least one person and the number of clusters. In the processing apparatus according to an aspect of the present invention the body movement determination unit may determine the presence or absence of the body movement of the at least one person within the measurement range for each of the plurality of periods, the person count estimation unit may estimate the number of the at least one person within the measurement range for each of the plurality of periods, and estimate that a maximum value of the number of clusters is the number of the at least one person within the measurement range for a period in which the absence is determined of the body movement of the at least one person within the measurement range among a continuous period in the plurality of periods, and the abnormality detection unit may determine that a period among the continuous period in which the absence is determined of the body movement of the at least one person within the measurement range and the number of clusters is smaller than the maximum value is a period in which the vital abnormality of the at least one person has occurred. In this manner, the user can determine that a vital abnormality of the at least one person within the measurement range and the period in which the vital abnormality has occurred.

The processing apparatus according to an aspect of the present invention may further include an abnormality detection unit configured to detect a vital abnormality of the at least one person within the measurement range. The body movement determination unit may determine the presence or absence of the body movement of the at least one person within the measurement range for each of the plurality of periods, the person count estimation unit may estimate the number of the at least one person within the measurement range for each of the plurality of periods, and estimate that a maximum value of the number of clusters is the number of the at least one person within the measurement range for a period in which the absence is determined of the body movement of the at least one person within the measurement range among a continuous period in the plurality of periods, and the abnormality detection unit may determine whether a vital abnormality has occurred in the at least one person within the measurement range based on the vital signal in a period immediately preceding a period among the continuous period in which the absence is determined of the body movement of the at least one person within the measurement range and the number of clusters is smaller than the maximum value. In this manner, the user can determine the vital abnormality of the at least one person within the measurement range.

Note that the present invention can be regarded as a processing method including at least some of the above-mentioned processes, a program for causing a computer to execute at least some of the above-mentioned processes, or a computer-readable recording medium on which such a program is recorded in a non-transitory manner. Each of the configurations and processes described above can be combined with one another to constitute the present invention, as long as no technical inconsistency arises.

### ADVANTAGEOUS EFFECTS OF INVENTION

According to the present invention, time-series data of the vital index can be calculated for each person.

### BRIEF DESCRIPTION OF THE DRAWINGS

[FIG. 1] FIG. 1 is a diagram schematically illustrating an exemplary configuration of a processing apparatus.
[FIG. 2] FIG. 2 is a diagram illustrating a schematic configuration of the processing apparatus.
[FIG. 3] FIG. 3 is a block diagram illustrating an example of the processing apparatus.
[FIG. 4] FIG. 4 is a block diagram illustrating a configuration of a function of a signal acquisition unit.
[FIG. 5] FIG. 5 is a flowchart illustrating an example of a processing flow of the processing apparatus.
[FIG. 6] FIG. 6A is an explanatory diagram of coordinates of a representative waveform of a clustered respiratory waveform group, and FIG. 6B is a diagram illustrating an example of each representative waveform of the clustered respiratory waveform group.
[FIG. 7] FIG. 7 is an explanatory diagram of an estimation process of the number of persons within a measurement range.
[FIG. 8] FIG. 8 is an explanatory diagram of an example of excluding low-reliability data from time-series data of a vital index.
[FIG. 9] FIG. 9 is an explanatory diagram of an example of a process of outputting time-series data of the respiratory index for each person within the measurement range.
[FIG. 10] FIG. 10 is an explanatory diagram of an example of a process of outputting time-series data of the respiratory index for each person within the measurement range.
[FIG. 11] FIG. 11 is a flowchart illustrating an example of a processing flow of determining whether there is a body movement of a person within a measurement range.
[FIG. 12] FIG. 12A is a diagram illustrating an amplitude intensity of a received signal from which a static component has been removed in a state where a person is at rest. FIG. 12B is a diagram illustrating an amplitude intensity of a received signal from which a static component has been removed in a state where a body movement of a person has occurred.
[FIG. 13] FIG. 13 is a flowchart illustrating an example of a processing flow of determining whether there is a possibility of a change in the estimated number of persons.
[FIG. 14] FIGS. 14A to 14C are explanatory diagrams of a process of determining a change in the number of persons.
[FIG. 15] FIGS. 15A to 15C are diagrams illustrating a signal intensity of a received signal from which a static component has been removed.
[FIG. 16] FIGS. 16A to 16D are diagrams illustrating results of clustering.

### DESCRIPTION OF EMBODIMENTS

Hereinafter, an application example and an embodiment will be described with reference to the drawings. The application example and embodiment described below are merely one aspect of the present application and are not intended to limit the scope of rights of the present application.

### <Application Example>

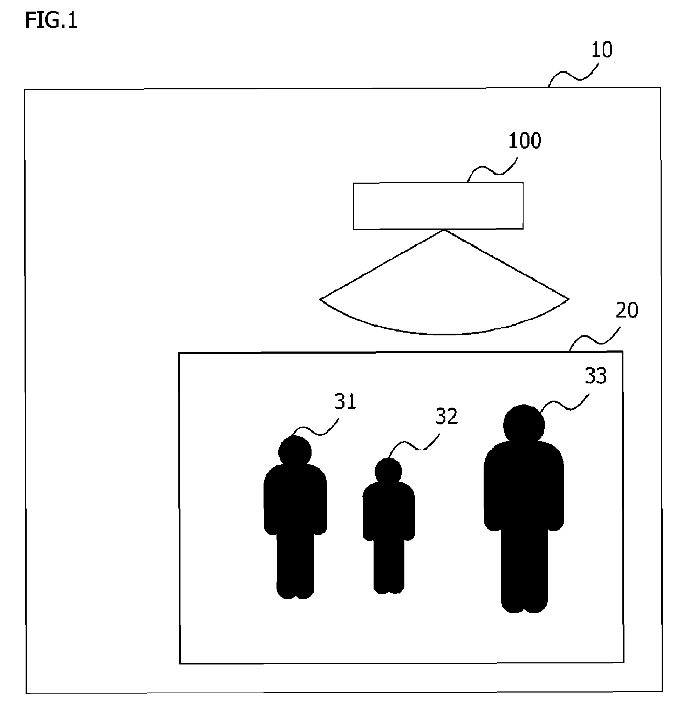

FIG. 1 is a diagram schematically illustrating an example of use of a processing apparatus 100 to which the present invention is applied. In the example of use illustrated in FIG. 1, three persons 31, 32 and 33 to be measured are positioned side by side on a bed 20 installed in a room 10. In addition, the processing apparatus 100 is disposed in the room 10. The processing apparatus 100 transmits signals to the persons 31, 32 and 33 to be measured on the bed 20, and performs so-called non-contact sensing. Examples of the frequency of the signal transmitted to the persons 31, 32, and 33 to be measured may be a frequency in the range of 30 GHz to 300 GHz used in millimeter-wave radar; however, other frequency bands such as light, radio waves, sound waves, or ultrasonic waves may also be adopted.

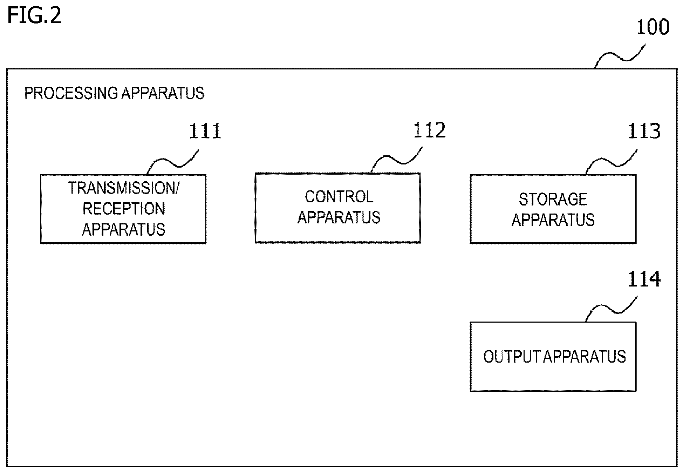

FIG. 2 is a diagram illustrating an exemplary configuration of the processing apparatus 100. The processing apparatus 100 includes a transmission/reception apparatus 111, a control apparatus 112, a storage apparatus 113, and an output apparatus 114. The transmission/reception apparatus 111 functions as a signal reception unit that receives a signal reflected by at least one person within the measurement range. For example, the transmission/reception apparatus 111 transmits and receives signals to and from the persons 31, 32 and 33 illustrated in FIG. 1. The measurement range of the processing apparatus 100 is, for example, a predetermined range on the bed 20 illustrated in FIG. 1, but is not limited to this predetermined range. The control apparatus 112 acquires a vital signal from a signal reflected by at least one person within the measurement range. In addition, the control apparatus 112 computes a vital index representing the vital state of at least one person within the measurement range based on the vital signal. The storage apparatus 113 stores various data such as signal data received by the transmission/reception apparatus 111, data used in processes executed by the control apparatus 112, and data generated in such processes. In accordance with the results of the processes executed by the control apparatus 112, the output apparatus 114 provides a notification to the user and/or outputs data related to the results of the processes to an external apparatus. Note that the output apparatus 114 may be configured to be able to output data related to the results of the processes to an external apparatus by various communication methods such as various types of wireless communication or wired communication.

The processing apparatus 100 performs non-contact sensing for at least one person within the measurement range by using a signal transmission/reception means such as a radio wave radar, an ultrasonic sensor, or an acoustic sensor to compute the vital index representing the vital state of the person and estimate the number of persons within the measurement range. The processing apparatus 100 calculates the time-series data of the vital index for each person, and when there is a possibility of a change in the estimated number of persons, the processing apparatus 100 separately calculates the time-series data of the vital index before it is determined that there is a possibility of a change in the estimated number of persons and the time-series data of the vital index after it is determined that there is a possibility of a change in the estimated number of persons. In this manner, according to the processing apparatus 100, the time-series data of the vital index corresponding to the number of persons before the number of persons has changed and the time-series data of the vital index corresponding to the number of persons after the number of persons has changed can be separately calculated for each person.

### <Description of Embodiments>

An embodiment of a technique of the present disclosure is described below. As an example, the present embodiment assumes a case where the processing apparatus 100 and the bed 20 are disposed in the room 10 as illustrated in FIG. 1, with a plurality of persons 31, 32 and 33 lying on the bed 20, and the vital index of each person to be measured is acquired by the processing apparatus 100. For example, the processing apparatus 100 may acquire the vital index of each person during sleep. Note here that the vital index to be acquired is assumed to be an index (respiratory index) related to the respiration of the person to be measured, but the vital index to be acquired may be an index (heart rate index) related to the heart rate.

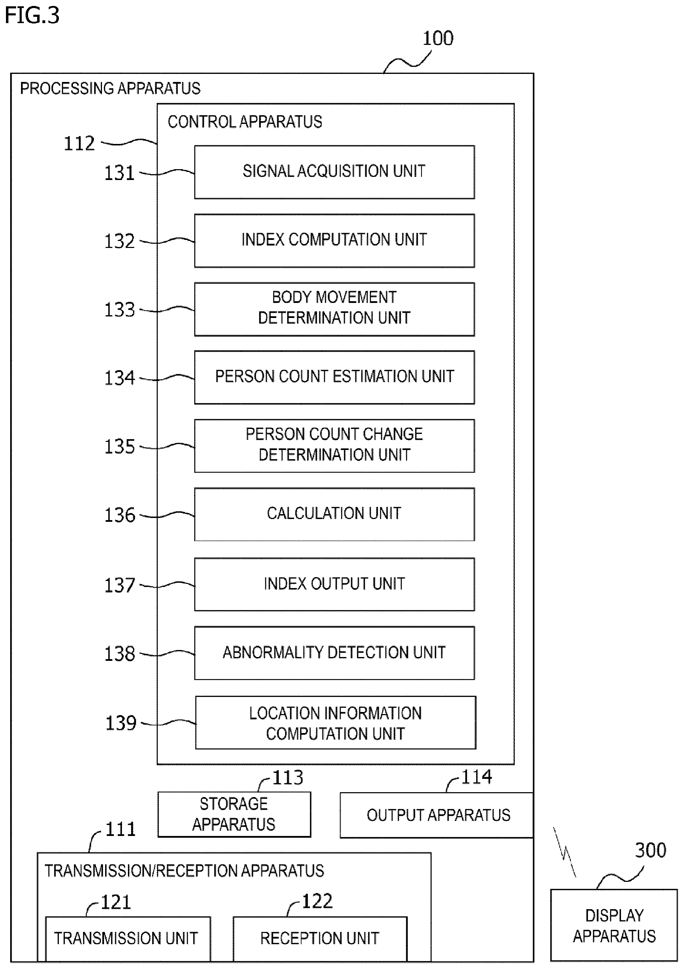

FIG. 3 is a block diagram illustrating an exemplary configuration of the processing apparatus 100 according to the embodiment. As illustrated in FIG. 3, in the processing apparatus 100, the transmission/reception apparatus 111 includes a transmission unit 121 that transmits a signal to a person within the measurement range, and a reception unit 122 that receives the signal reflected by the person within the measurement range. The control apparatus 112 includes a signal acquisition unit 131, an index computation unit 132, a body movement determination unit 133, a person count estimation unit 134, a person count change determination unit 135, a calculation unit 136, an index output unit 137, an abnormality detection unit 138, and a location information computation unit 139.

The control apparatus 112, which includes a central processing unit (CPU), a random access memory (RAM), a read only memory (ROM) and the like, controls each unit in the processing apparatus 100 and performs various processes and the like. The storage apparatus 113 stores various data and the like used in programs executed at the control apparatus 112 and processes executed at the control apparatus 112. For example, the storage apparatus 113 is an auxiliary storage apparatus such as a hard disk drive (HDD) and a solid state drive (SSD). The storage apparatus 113 may be implemented by a detachable storage medium. The output apparatus 114 outputs data generated by the control apparatus 112 to a display apparatus 300. Note that the data generated by the control apparatus 112 may be stored in the storage apparatus 113 and output from the output apparatus 114 to the display apparatus 300 at a given timing. The output apparatus 114 may be a communication interface implemented by a communication apparatus such as a network card, for example.

In addition, in the present embodiment, the processing apparatus 100 and the display apparatus 300 are separate apparatuses, but the processing apparatus 100 may be configured integrally with the display apparatus 300. Not all components of the processing apparatus 100 illustrated in FIG. 3 may not be essential, and components of the processing apparatus 100 may be added or omitted as necessary. In addition, at least some the functions of the processing apparatus 100 may be implemented by a computer on the cloud, or may be a microcomputer such as a programmable logic controller (PLC) and a single board computer.

The signal acquisition unit 131 acquires a vital signal from a signal reflected by at least one person within the measurement range. The index computation unit 132 computes a vital index representing the vital state of at least one person within the measurement range based on the vital signal. The body movement determination unit 133 determines whether there is a body movement of the person within the measurement range. The person count estimation unit 134 estimates the number of persons (person count) within the measurement range. The person count change determination unit 135 determines whether there is a possibility of a change in the estimated number of persons. Specifically, the person count change determination unit 135 determines whether there is a possibility of an increase or decrease in the number of persons within the measurement range. The calculation unit 136 calculates the time-series data of the vital index for each person within the measurement range. When it is determined that there is a possibility of a change in the estimated number of persons, the calculation unit 136 separately calculates the time-series data of the vital index before it is determined that there is a possibility of a change in the estimated number of persons and the time-series data of the vital index after it is determined that there is a possibility of a change in the estimated number of persons. The index output unit 137 outputs the time-series data of the vital index for each person within the measurement range. The abnormality detection unit 138 detects the vital abnormality of at least one person within the measurement range. The location information computation unit 139 identifies the position of the person within the measurement range, and computes the location information of the person associated with the vital index for each person.

The processing apparatus 100 executes various processes in predetermined time slots. Accordingly, the signal acquisition unit 131 acquires the vital signal in each of a plurality of periods. The index computation unit 132 computes the vital index of each of the plurality of periods. The body movement determination unit 133 determines whether there is a body movement of the person within the measurement range in each of the plurality of periods. The person count estimation unit 134 estimates the number of persons within the measurement range in each of the plurality of periods. The person count change determination unit 135 determines whether there is a possibility of a change in the estimated number of persons in each of the plurality of periods. The abnormality detection unit 138 detects the vital abnormality of at least one person within the measurement range in each of the plurality of periods. The location information computation unit 139 identifies the position of the person within the measurement range in each of the plurality of periods, and computes the location information of the person associated with the vital index for each person.

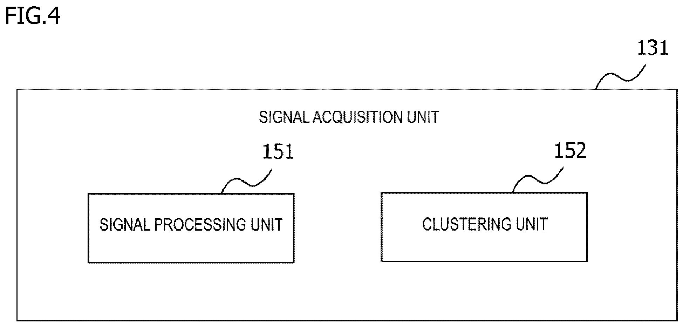

FIG. 4 is a block diagram illustrating a configuration of a function of the signal acquisition unit 131. The function of the signal acquisition unit 131 is composed of a signal processing unit 151 and a clustering unit 152. The signal processing unit 151 performs signal processing on a signal received by the reception unit 122, and acquires a vital waveform group based on a vital signal extracted from the signal received by the reception unit 122. Here, the vital signal is, for example, a respiratory signal, but may also be a heart rate signal. In addition, the vital waveform group is, for example, a respiratory waveform group, but may also be a heart rate waveform group. The clustering unit 152 executes a process of clustering the vital waveform group, and acquires the number of clusters of the clustered vital waveform group, the waveform of each vital waveform group and the coordinates (location information) of the waveform.

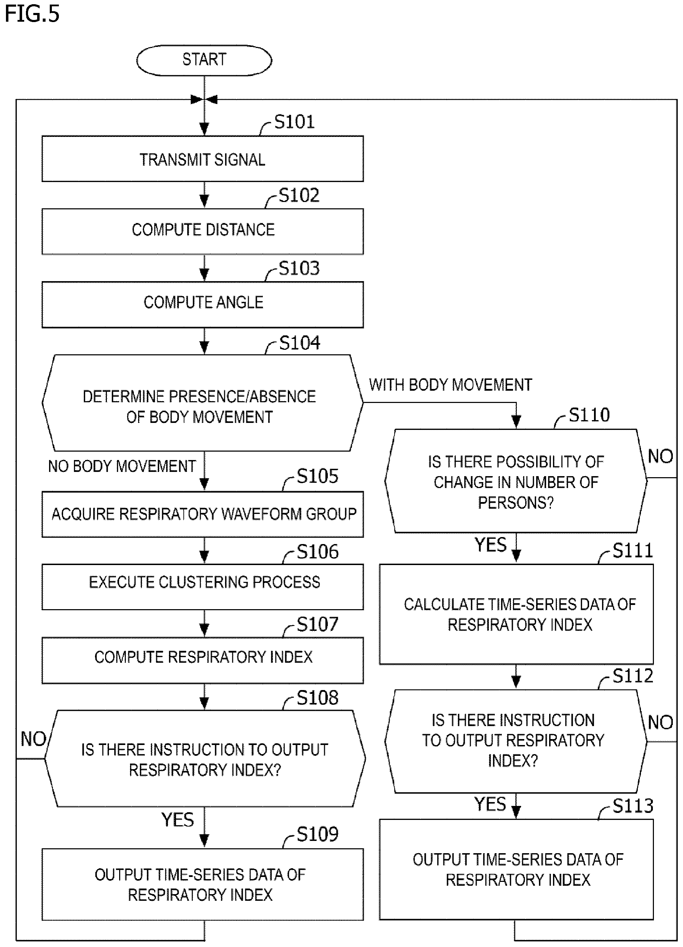

FIG. 5 is a flowchart illustrating an example of a processing flow of the processing apparatus 100. As an example, the process of FIG. 5 is executed by instructing the processing apparatus 100 to start the processing flow of FIG. 5. At step S101, the transmission unit 121 transmits a signal into the measurement range. For example, the transmission unit 121 may transmit a chirp signal. The frequency band of the chirp signal transmitted by the transmission unit 121 and the transmission scheme such as up-chirp or down-chirp may be appropriately set. Here, as an example, it is assumed that the frequency modulated continuous wave (FMCW) scheme is used, with a transmission/reception sampling period of approximately 100 µs, and an array of eight-channel antennas is employed. The reception unit 122 receives a signal reflected by at least one person, stationary object or the like within the measurement range.

At step S102, the signal processing unit 151 performs signal processing on an IF signal obtained from the difference between the chirp signal transmitted by the transmission unit 121 and the signal received by the reception unit 122, and computes the distance from the transmission/reception apparatus 111 (the processing apparatus 100) to the position where the signal is reflected (measurement position). More specifically, the signal processing unit 151 computes the distance from the transmission/reception apparatus 111 to the measurement position based on different frequency spectra obtained by performing Fourier Transform (FFT) on the IF signal after AD conversion.

At step S103, the signal processing unit 151 performs signal processing on the IF signal, and computes the azimuth (angle) of the measurement position with respect to the transmission/reception apparatus 111 (the processing apparatus 100). More specifically, the signal processing unit 151 computes the angle (arrival azimuth) from the phase difference among the received signals of a plurality of antennas of the reception unit 122. At step S104, the body movement determination unit 133 determines whether there is a body movement of the person within the measurement range. When it is determined that there is no body movement of the person within the measurement range, the process proceeds to step S105. When it is determined that there is a body movement of the person within the measurement range, the process proceeds to step S110.

At step S105, the signal processing unit 151 acquires a respiratory waveform group based on a respiratory signal extracted through the signal processing on the signal received by the reception unit 122. The signal received by the reception unit 122 contains signals not resulting from the respiration motion. Examples of the signals not resulting from the respiration motion include signals from stationary objects such as beds or walls, subtle movements of a person, vibrations from a fan, movements of pets or robots, signals mixed from a plurality of objects, and noise components. The signal processing unit 151 removes only the signal resulting from the respiration motion (the respiratory signal) from the signal received by the reception unit 122 by removing the signals not resulting from the respiration motion from the signal received by the reception unit 122. The signal processing unit 151 performs exclusion of signals not resulting from respiratory motion and extraction of respiratory signals based on characteristics such as the signal intensity of the reflected wave, phase fluctuation amount, amplitude fluctuation amount, phase periodicity, and frequency.

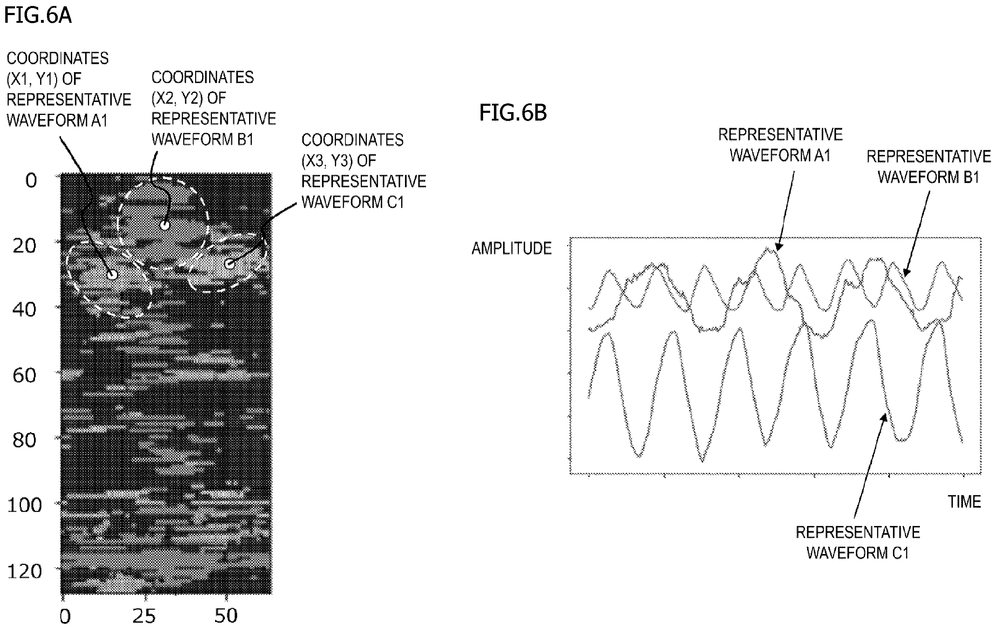

At step S106, the clustering unit 152 clusters the respiratory waveform group based on the feature computed from the vital signal, and acquires the number of clusters of the clustered respiratory waveform group, the representative waveform of each respiratory waveform group and the coordinate of the representative waveform. The clustering unit 152 may cluster the respiratory waveform group by using the k means clustering algorithm. The clustering unit 152 may perform clustering with features computed from the respiratory waveform group as the input. The feature is the frequency, phase and coordinate information (distance, azimuth) and the like of the vital signal, for example. In addition, the clustering unit 152 may cluster the respiratory waveform group by using algorithms such as the Gaussian mixture model (GMM), X-means, and variational Bayesian GMM (VBGMM). FIG. 6A is an explanatory diagram of coordinates of a representative waveform of a clustered respiratory waveform group. The abscissa in FIG. 6A indicates azimuth, and the ordinate in FIG. 6A indicates distance. FIG. 6A illustrates coordinates (X1, Y1) of a representative waveform A1, coordinates (X2, Y2) of a representative waveform B1, and coordinates (X3, Y3) of a representative waveform C1. FIG. 6B is a diagram illustrating an example of each representative waveform of the clustered respiratory waveform group. The ordinate in FIG. 6B indicates amplitude, and the abscissa in FIG. 6B indicates time.

At step S107, the index computation unit 132 computes the respiratory index representing the respiratory state of at least one person within the measurement range based on the respiratory signal. The index computation unit 132 computes the respiratory index corresponding to one slot. One slot is 20 seconds (250 frames), for example.

At step S108, the index output unit 137 determines whether there is an instruction to output the respiratory index. When there is an instruction to output the respiratory index (S108: YES), the process proceeds to step S109. When there is no instruction to output the respiratory index (S108: NO), the process is returned to step S101. The user may input an instruction to output the respiratory index to the processing apparatus 100 by operating the processing apparatus 100.

At step S109, the calculation unit 136 computes the time-series data of the respiratory index for each person within the measurement range, and the index output unit 137 outputs the time-series data of the respiratory index for each person within the measurement range. Thereafter, the process is returned to step S101. The time-series data of the respiratory index is data in which respiratory indices of a plurality of slots are arranged in a time-series manner, and data representing the time trend of the respiratory index. In addition, the calculation unit 136 may calculate the time-series data of the respiratory index at a given timing, or calculate the time-series data of the respiratory index based on schedule information set in advance. The index output unit 137 may output the time-series data of the respiratory index at a given timing, or output the time-series data of the respiratory index based on schedule information set in advance.

At step S110, the person count estimation unit 134 estimates the number of persons within the measurement range, and the person count change determination unit 135 determines whether there is a possibility of a change in the estimated number of persons. When it is determined that there is a possibility of a change in the estimated number of persons (S110: YES), the process proceeds to step S111. When it is determined that there is no possibility of a change in the estimated number of persons (S110: NO), the process is returned to step S101.

At step S111, the calculation unit 136 calculates the time-series data of the respiratory index for each person within the measurement range. In this case, the calculation unit 136 separately calculates the time-series data of the respiratory index before it is determined that there is a possibility of a change in the number of persons and the time-series data of the respiratory index after it is determined that there is a possibility of a change in the number of persons. In this manner, the time-series data of the vital index corresponding to the number of persons before the number of persons has changed and the time-series data of the vital index corresponding to the number of persons after the number of persons has changed can be separately calculated for each person. After the process of step S111 has been performed, the process is returned to step S101.

At step S112, the index output unit 137 determines whether there is an instruction to output the respiratory index. When there is an instruction to output the respiratory index (S112: YES), the process proceeds to step S113. When there is no instruction to output the respiratory index (S112: NO), the process is returned to step S101. The user may input an instruction to output the respiratory index to the processing apparatus 100 by operating the processing apparatus 100.

At step S113, the index output unit 137 outputs the time-series data of the respiratory index for each person within the measurement range. In this case, the index output unit 137 separately outputs the time-series data of the respiratory index before it is determined that there is a possibility of a change in the number of persons and the time-series data of the respiratory index after it is determined that there is a possibility of a change in the number of persons. In this manner, the time-series data of the vital index corresponding to the number of persons before the number of persons has changed and the time-series data of the vital index corresponding to the number of persons after the number of persons has changed can be separately output for each person.

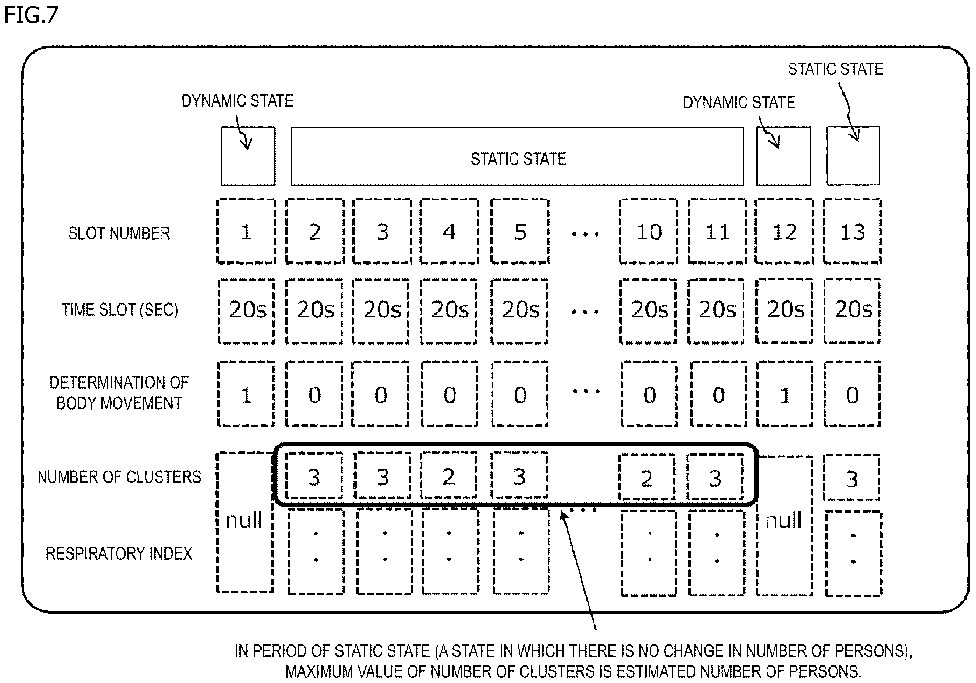

FIG. 7 is an explanatory diagram of an estimation process of the number of persons within a measurement range. Each slot (time slot) of FIG. 7 is 20 seconds. In the first slot (slot number 1) and the twelfth slot (slot number 12), it is determined that there is a body movement of the person within the measurement range (body movement determination = 1) at step S104 of the processing flow of FIG. 5. Accordingly, in each of the periods of the first slot and twelfth slot, at least one person within the measurement range is in a dynamic state. In the second to eleventh and thirteenth slots (slot numbers 2 to 11 and 13), it is determined that there is no body movement of the person within the measurement range (body movement determination = 0) at step S104 of the processing flow of FIG. 5. Accordingly, in each of the periods of the second to eleventh and thirteenth slots, all persons within the measurement range are in a static state. In FIG. 7, the maximum value of the number of clusters of the second to eleventh and thirteenth slots is "3".

In a period in which the person within the measurement range body is not in a movement state and it is determined that there is no movement of the person within the measurement range, it can be estimated that there is no change in the number of persons within the measurement range, and therefore the maximum value of the result of the clustering process of the respiratory waveform group can be determined to be the correct number of persons within the measurement range. It can be estimated that when there is the largest number of vital indices (respiratory indices) in the period of the static state (a state in which there is no change in the number of persons), the vital indices corresponding to the actual number of persons are measured, and that in other cases, the vital signals corresponding to the number of persons could not be acquired due to reasons such as the vital signals not meeting the extraction criteria or deterioration of the S/N ratio. The person count estimation unit 134 estimates the number of persons within the measurement range based on the maximum value of the number of clusters in the period of the static state (a state in which there is no change in the number of persons). Specifically, for the period where it is determined that there is no body movement of the person within the measurement range among a continuous period in the plurality of periods, the person count estimation unit 134 estimates that the maximum value of the number of clusters is the number of persons within the measurement range. In the example illustrated in FIG. 7, the person count estimation unit 134 estimates that the number of persons within the measurement range in the static state period is "3".

In FIG. 7, the number of clusters in the fourth slot and tenth slot is "2", which does not match the maximum value of the number of clusters in the static state period. In such a case, it is possible that a person in a state different from a normal respiratory state, such as an apnea state, is present within the measurement range. Therefore, in the period in which the number of clusters is smaller than the maximum value of the number of clusters in the static state period, it is highly possible that the person within the measurement range has been in an abnormal state such as apnea or coughing. It is preferable to detect the period in which the person within the measurement range may have been in an abnormal state such as apnea or coughing. In view of this, in the present embodiment, the abnormality detection unit 138 detects the vital abnormality of at least one person within the measurement range based on the estimated number of persons and number of clusters. When the estimated number of persons (the maximum value of the number of clusters) and the number of clusters do not coincide with each other, the abnormality detection unit 138 determines that a vital abnormality has occurred in at least one person within the measurement range. Then, the abnormality detection unit 138 determines that a period in which it is determined that there is no body movement of the person within the measurement range and the number of clusters is smaller than the maximum value of the number of clusters among a continuous period in the plurality of periods is a period in which a vital abnormality has occurred in the person within the measurement range. In this manner, the user can determine that a vital abnormality of the person within the measurement range and the period in which the vital abnormality has occurred.

The abnormality detection unit 138 may detect the vital abnormality of at least one person within the measurement range based on a change in the vital signal of the slot immediately preceding the slot where the estimated number of persons and number of clusters do not coincide with each other. For example, the abnormality detection unit 138 may determine whether a vital abnormality has occurred in at least one person within the measurement range by detecting a gradual decrease in respiratory rate, a gradual decrease in amplitude value of the vital signal or the like based on a change in the vital signal. The abnormality detection unit 138 may determine whether a characteristic that coincides with the characteristic of the movement of the body surface at the time of the occurrence of the vital abnormality has been detected from the received signal of the spatial region where the respiratory signal has been successfully acquired based on the slot where the estimated number of persons and number of clusters do not coincide with each other, and the immediately preceding slot. The abnormality detection unit 138 may determine whether a vital abnormality has occurred in at least one person within the measurement range based on the result of the determination whether a characteristic that coincides with the characteristic of the movement of the body surface at the time of the occurrence of the vital abnormality has been detected from the received signal of the spatial region where the respiratory signal has been successfully acquired. In this manner, the abnormality detection unit 138 may determine whether a vital abnormality has occurred in at least one person within the measurement range based on the vital signal in the period immediately preceding the period in which it is determined that there is no body movement of the person within the measurement range and the number of clusters is smaller than the maximum value of the number of clusters among a continuous period in the plurality of periods. In this manner, the user can determine the vital abnormality of the person within the measurement range.

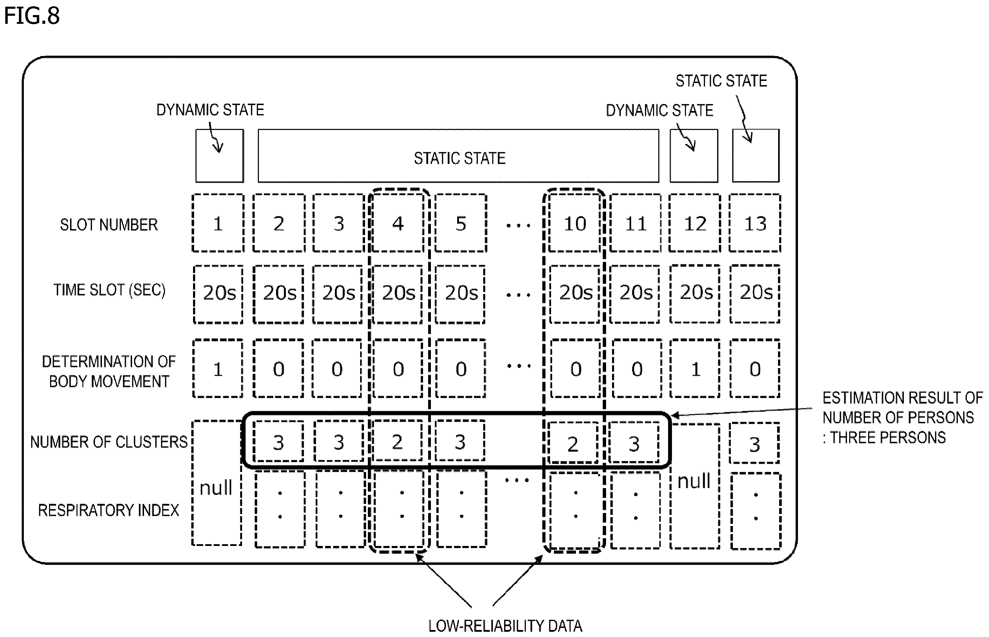

FIG. 8 is an explanatory diagram of an example of excluding low-reliability data from time-series data of a vital index. The slot numbers, slots (time slots), body movement determination, the number of clusters and the respiratory index of FIG. 8 are the same as those of FIG. 7. In the fourth slot (slot number 4) and tenth slot (slot number 10), the number of clusters is "2", and the maximum value of the number of clusters in the static state period is "3". Since the person count estimation unit 134 estimates the number of persons within the measurement range based on the maximum value of the number of clusters in the static state period, the estimation result of the number of persons in the periods of the first to eleventh slots is three persons. In the period of the fourth slot and the period of the tenth slot, the respiratory indices corresponding to the number of persons estimated by the person count estimation unit 134 have not been computed, and therefore the respiratory index data in the period of the fourth slot and the period of the tenth slot is low-reliability data. It is preferable to exclude the low-reliability data from the time-series data of the vital index. In view of this, in the present embodiment, the calculation unit 136 calculates the time-series data of the vital index for each person by excluding the period in which the vital indices (e.g., respiratory indices) corresponding to the estimated number of persons have not been computed in the period in which it is determined that there is no body movement of the person within the measurement range (static state period) among the plurality of periods. In this manner, the low-reliability data is excluded from the time-series data of the vital index, and thus the reliability of the time-series data of the vital index is improved.

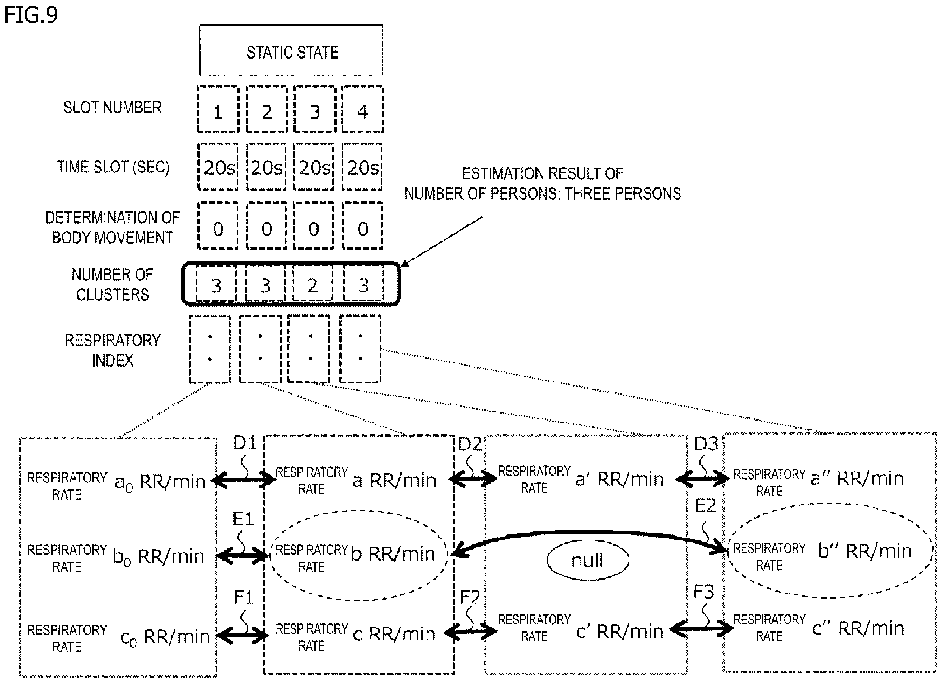

FIG. 9 is an explanatory diagram of an example of a process of outputting time-series data of the respiratory index for each person within the measurement range. Each slot (time slot) of FIG. 9 is 20 seconds. The respiratory index illustrated in FIG. 9 includes the respiratory rate. The number of clusters is "3" in the periods of the first, second and fourth slots (slot numbers 1, 2 and 4), and the number of clusters in the period of the third slot (slot number 3) is "2". The person count estimation unit 134 estimates the number of persons within the measurement range based on the maximum value of the number of clusters in the static state period, and therefore the estimation result of the number of persons in the periods of the first to fourth slots is three persons. In the periods of the first, second and fourth slots, the respiratory rates corresponding to the estimated number of persons have been computed. In the period of the third slot, the respiratory rates corresponding to the estimated number of persons have not been computed. In this case, it is determined that the respiratory signal of the targeted person does not meet the extraction condition although the targeted person is present, and the respiratory rate corresponding to one person is not computed. Therefore, in the period of the third slot, the respiratory rates corresponding to the two persons are computed, and the computation result of the respiratory rate corresponding to one person is null.

The calculation unit 136 compares the respiratory rates of the periods of the respective slots, and determines the correspondence relationship of the respiratory rates of the periods of the respective slots for each person. The respiratory rates of adjacent slots of the same person are unlikely to change abruptly. In view of this, the calculation unit 136 determines a combination that has a minimum difference between the respiratory rates of adjacent slots. In the example illustrated in FIG. 9, the calculation unit 136 determines the combination of respiratory rates associated by arrows D1 to D3, the combination of respiratory rates associated by arrows E1 to E2, and the combination of respiratory rates associated by arrows F1 to F3. In this manner, the calculation unit 136 determines the combination of respiratory indices for each person by associating respiratory indices for each person based on the respiratory index value of each of the plurality of periods, and calculates the time-series data of the respiratory index for each person based on the combination of respiratory indices. While known methods cannot calculate the time-series data of the vital index for each person, the present embodiment can calculate the time-series data of the vital index for each person. When analyzing the time-series data of the vital index, the vital index can be easily determined for each person. Note that the calculation processing of the time-series data of the respiratory index is also applicable to the calculation processing of the time-series data of the pulse index and the calculation processing of the time-series data of the vital index.

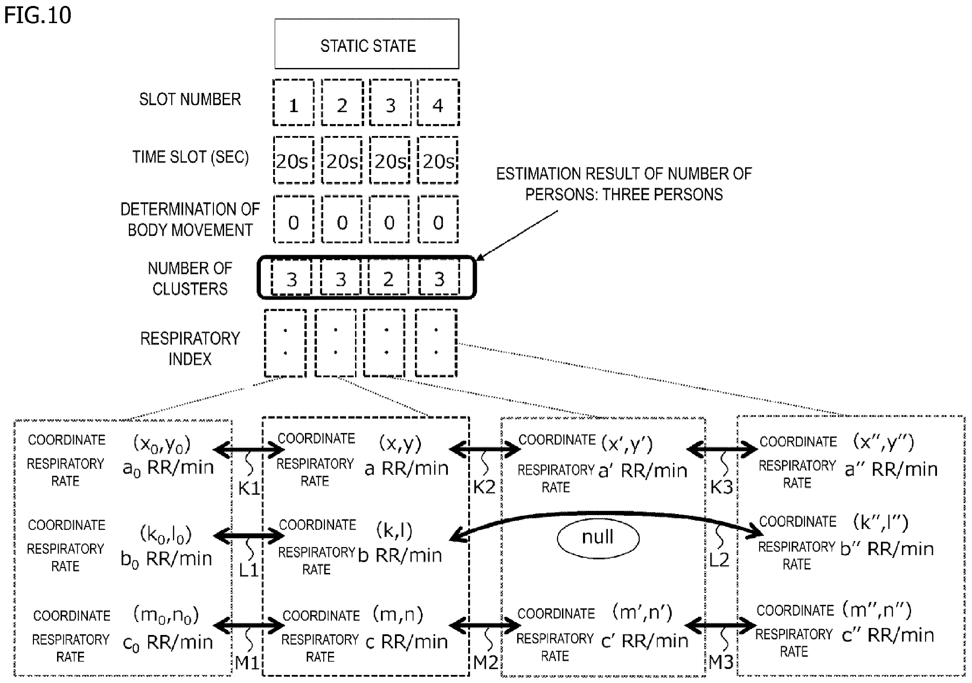

FIG. 10 is an explanatory diagram of an example of a process of calculating time-series data of the respiratory index for each person within the measurement range. Each slot (time slot) of FIG. 10 is 20 seconds. The respiratory index illustrated in FIG. 10 includes the respiratory rate and spatial coordinates (distance and azimuth). The coordinates included in the respiratory index may be coordinates of representative waveforms of a clustered respiratory waveform group. In the periods of the first, second and fourth slots, the coordinates and the respiratory rates corresponding to the estimated number of persons are computed. In the period of the third slot, the coordinates and the respiratory rates corresponding to the estimated number of persons are not computed. In the period of the third slot, the coordinates of the respiratory rates corresponding to the two persons are computed, and the computation result of coordinates and the respiratory rate corresponding to one person is null.

The calculation unit 136 compares the coordinates of the periods of the respective slots, and determines the correspondence relationship of the coordinates of the periods of the respective slots for each person. The calculation unit 136 determines a combination that has a minimum distance (coordinate-to-coordinate distance) between the coordinates of adjacent two slots. In the example illustrated in FIG. 10, the calculation unit 136 stores the combination of respiratory indices associated by arrows K1 to K3, the combination of respiratory indices associated by arrows L1 to L2, and the combination of respiratory indices associated by arrows M1 to M3. In this manner, the calculation unit 136 determines the combination of respiratory indices for each person by associating respiratory indices for each person based on the coordinates (location information) of the plurality of periods, and calculates the time-series data of the respiratory index for each person based on the combination of respiratory indices. In this manner, the time-series data of the vital index for each person within the measurement range can be correctly calculated. When analyzing the time-series data of the vital index, the vital index can be easily determined for each person. Note that the calculation processing of the time-series data of the respiratory index is also applicable to the calculation processing of the time-series data of the pulse index and the calculation processing of the time-series data of the vital index.

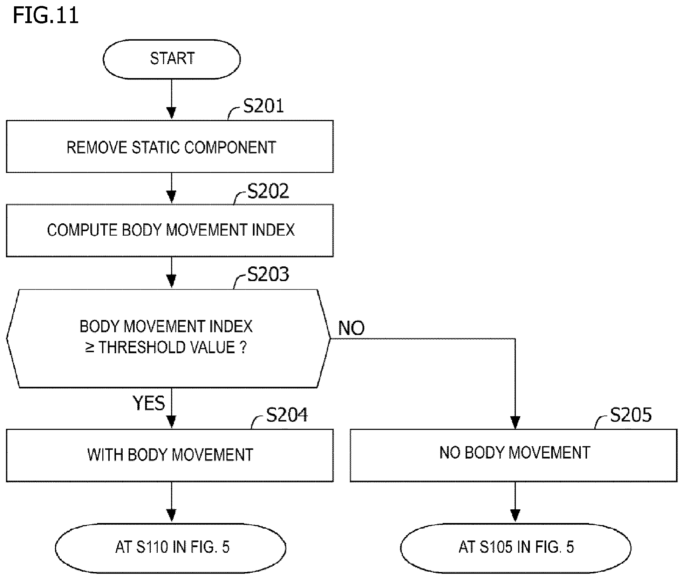

FIG. 11 is a flowchart illustrating an example of a processing flow of determining whether there is a body movement of a person within a measurement range. The processing flow of FIG. 11 is executed at step S104 of FIG. 5. At step S201, the signal processing unit 151 removes a static component from a received signal by performing signal processing on a signal received by the reception unit 122. The signal processing unit 151 may perform time-differential processing between adjacent frames, or time-averaged differential processing of signals within a slot from the signal of each frame, for example. At step S202, the signal processing unit 151 computes a body movement index (amplitude value) based on a signal from which a static component has been removed.

At step S203, the body movement determination unit 133 determines whether the body movement index is not smaller than a threshold value. When the body movement index is equal to or greater than the threshold value (S203: YES), the process proceeds to step S204. At step S204, the body movement determination unit 133 determines that there is a body movement of a person within a measurement range, and the process proceeds to step S110 in FIG. 5. When the body movement index is smaller than the threshold value (S203: NO), the process proceeds to step S205. At step S205, the body movement determination unit 133 determines that there is no body movement of a person within a measurement range, and the process proceeds to step S105 in FIG. 5.

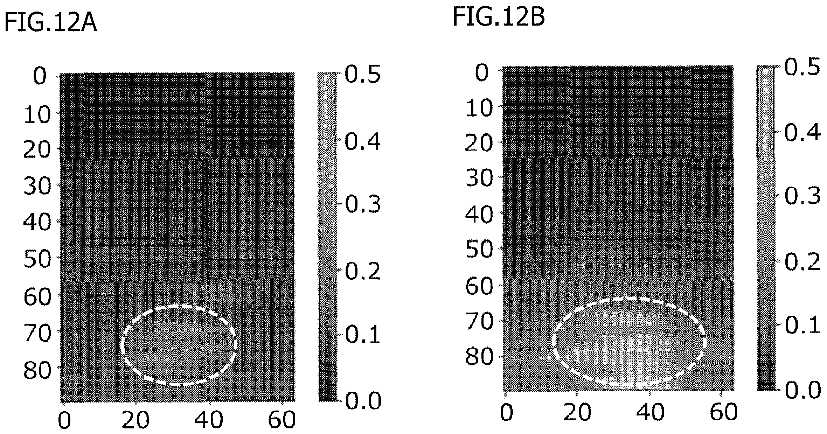

With reference to FIGS. 12A and 12B, an example of a process of determining whether there is a body movement of the person within the measurement range is described below. FIG. 12A is a diagram illustrating an amplitude intensity of a received signal from which a static component has been removed in a state where a person is at rest. FIG. 12B is a diagram illustrating an amplitude intensity of a received signal from which a static component has been removed in a state where a body movement of a person has occurred. The abscissa in FIGS. 12A and 12B indicates azimuth, and the ordinate in FIGS. 12A and 12B indicates distance. In a state where a body movement of the person has occurred, the amplitude value of the position where a person is present improves in comparison with a state where a person is at rest. The body movement determination unit 133 may determine whether there is a body movement of the person within the measurement range based on a change in amplitude value. In addition, in a state where a body movement of the person has occurred, the range where the amplitude value is strong is wider in comparison with a state where a person is at rest. The body movement determination unit 133 may determine whether there is a body movement of the person within the measurement range based on the degree of expansion of the range where the amplitude value is strong.

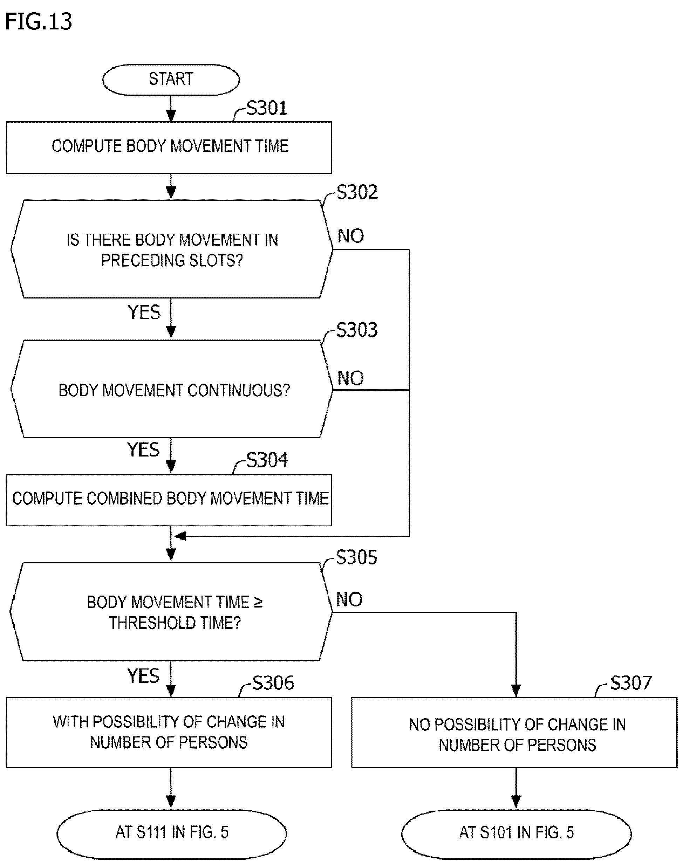

FIG. 13 is a flowchart illustrating an example of a processing flow of determining whether there is a possibility of a change in the estimated number of persons. The processing flow of FIG. 13 is executed at step S110 of FIG. 5. At step S301, the person count change determination unit 135 computes (measures) the body movement time. The body movement time is a time (elapsed time) from a timing when a body movement of a person within a measurement range is detected to a timing when the body movement of the person within the measurement range is no longer detected, for example.

At step S302, the person count change determination unit 135 determines whether there is a body movement of a person within a measurement range in preceding slots (slots preceding the process target slot). When there is a body movement of a person within a measurement range in preceding slots (S302: YES), the process proceeds to S303. When there is no body movement of a person within a measurement range in preceding slots (S302: NO), the process proceeds to S305.

At step S303, the person count change determination unit 135 determines whether the body movement of the person within the measurement range is continuous. More specifically, the person count change determination unit 135 determines whether the body movement of the person within the measurement range in the preceding slots and the body movement of the person within the measurement range in the process target slot are in a continuous relationship. When the body movement of the person within the measurement range in the preceding slots and the body movement of the person within the measurement range in the process target slot are in a continuous relationship, the person count change determination unit 135 determines that the body movement of the person within the measurement range is continuous. When the body movement of the person within the measurement range is continuous (S303: YES), the process proceeds to step S304. When the body movement of the person within the measurement range in the preceding slots and the body movement of the person within the measurement range in the process target slot are not in a continuous relationship, the person count change determination unit 135 determines that the body movement of the person within the measurement range is not continuous. When the body movement of the person within the measurement range is not continuous (S303: NO), the process proceeds to step S305.

At step S304, the person count change determination unit 135 computes a combined body movement time by summing the body movement time in the preceding slots and the body movement time in the process target slot. At step S305, the person count change determination unit 135 determines whether the body movement time is not shorter than a threshold time. When the body movement time is equal to or longer than the threshold time (S305: YES), the process proceeds to step S306. When the body movement time is shorter than the threshold value (S305: NO), the process proceeds to step S307. When the combined body movement time has been computed at step S304, the person count change determination unit 135 determines whether the combined body movement time is not shorter than a threshold time. When the combined body movement time is equal to or longer than the threshold time (S305: YES), the process proceeds to step S306. When the combined body movement time is shorter than the threshold time (S305: NO), the process proceeds to step S307.

At step S306, the person count change determination unit 135 determines that there is a possibility of a change in the estimated number of persons, and the process proceeds to step S111 in FIG. 5. At step S307, the person count change determination unit 135 determines that there is no possibility of a change in the estimated number of persons, and the process is returned to step S101 of FIG. 5. By determining whether there is a possibility of a change in the estimated number of persons based on the body movement time of the person, whether there is a possibility of a change in the estimated number of persons can be simply determined.

The threshold time may be determined based on the time during which the person within the measurement range can move to the outside of the measurement range. For example, the threshold time may be determined based on the times described in the following (1) and (2).
(1) Time for a person to get up from the bed (time from when the person gets into the bed to when they lie down)
(2) Time for a person to move from within the measurement range to the outside of the measurement range (time from when the person moves from the outside of the measurement range to the bed within the measurement range) Assuming that the shortest distance from the position of the person within the measurement range to the outside of the measurement range is 0.5 m, and the movement speed of the person is 1 m/sec, the time for (1) is 5 seconds and the time for (2) is 0.5 seconds, and therefore, the threshold time may be set to 5.5 seconds.

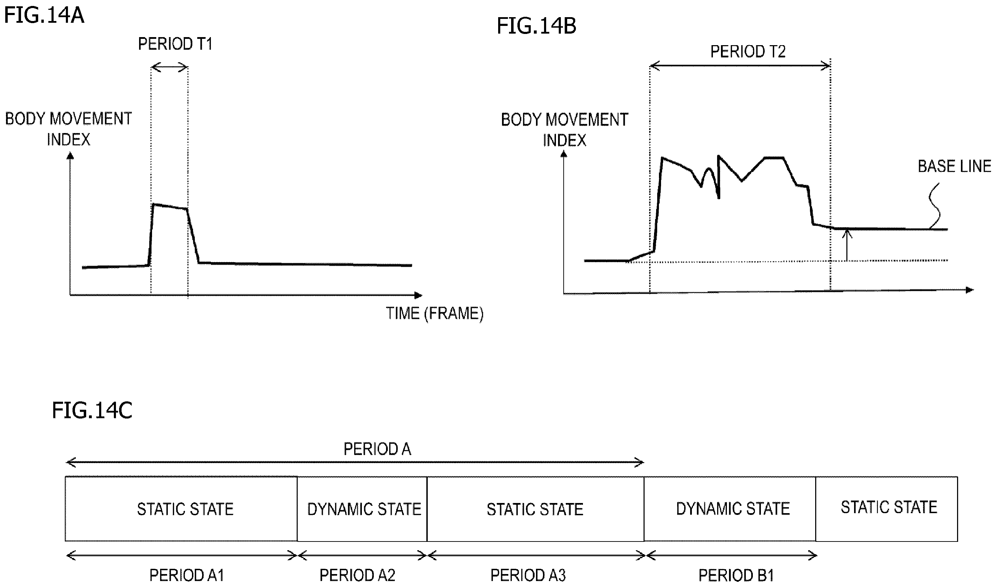

With reference to FIGS. 14A to 14C, an example of a process of determining a change in the number of persons by the person count change determination unit 135 is described below. FIGS. 14A to 14C are explanatory diagrams of a process of determining a change in the number of persons. The ordinate in FIGS. 14A and 14B indicates body movement index, and the abscissa in FIGS. 14A and 14B indicates time (frames). The body movement index is an index that indicates the magnitude of the body movement. The person count change determination unit 135 may compute the body movement index based on the amplitude information of a signal received by the reception unit 122 and the like. A period T1 illustrated in FIG. 14A where the body movement index has increased and a period T2 illustrated in FIG. 14B where the body movement index has increased are periods in which a body movement of a person within a measurement range has occurred. The period T1 and the period T2 may be the elapsed time from a timing when a body movement of a person within a measurement range is detected to a timing when the body movement of the person within the measurement range is no longer detected. The period T1 illustrated in FIG. 14A corresponds to a dynamic state period A2 illustrated in FIG. 14C, and the period (T2) illustrated in FIG. 14B corresponds to a dynamic state period (B1) illustrated in FIG. 12C.

When the period (T1) is shorter than the threshold time (specified time), the person count change determination unit 135 determines that there is no possibility of a change in the estimated number of persons. As illustrated in FIG. 12C, the dynamic state period (A2) corresponding to the period (T1) is a period between a static state period (A1) and a static state period (A3). The person count change determination unit 135 estimates that a period A including the period (A1), the period (A2) and the period (A3) is a period in which there is no change in the number of persons within the measurement range.

In the case where the period (T2) is longer than the threshold time, the person count change determination unit 135 may determine that there is a possibility of a change in the estimated number of persons. In addition, as illustrated in FIG. 12B, in the case where there is a change in the base line of the body movement index, it is possible that the person within the measurement range has moved. In the case where there is a change in the base line of the body movement index, the person count change determination unit 135 may determine that there is a possibility of a change in the estimated number of persons.

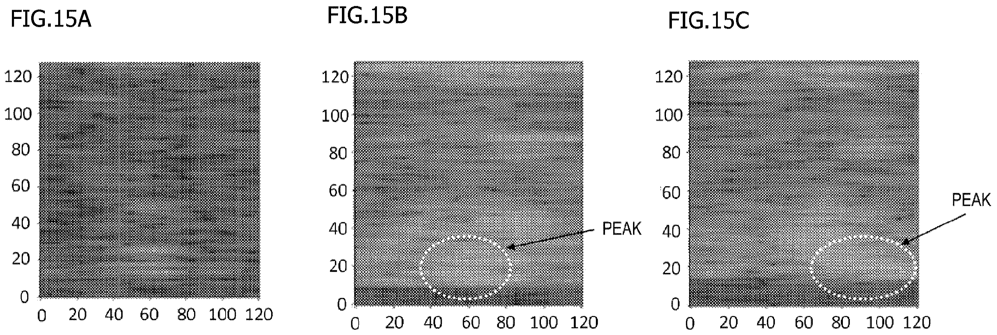

With reference to FIGS. 15A to 15C, an example of a process of determining a change in the number of persons by the person count change determination unit 135 is described below. FIG. 15A is a diagram illustrating a signal intensity of a received signal from which a static component in a state where a person is at rest has been removed. FIG. 15B is a diagram illustrating a signal intensity of a received signal from which a static component in a state where a body movement of a person has occurred has been removed. FIG. 15C is a diagram illustrating a signal intensity of a received signal in a state where the person is moving. The abscissa in FIGS. 15A to 15C indicates azimuth, and the ordinate in FIGS. 15A to 15C indicates distance.

In a state where a body movement of a person has occurred, there is a signal peak as illustrated in FIG. 15B, and in a state where the person is moving as illustrated in FIG. 15C, there is a change in the position of the signal peak generated by the body movement of the person. The person count change determination unit 135 may determine that there is a possibility of a change in the estimated number of persons based on a change in the position of the signal peak. For example, the person count change determination unit 135 may determine whether the person has moved to the outside of the measurement range from within the measurement range by tracking the change in the position of the signal peak, and determine whether there is a possibility of a change in the estimated number of persons.

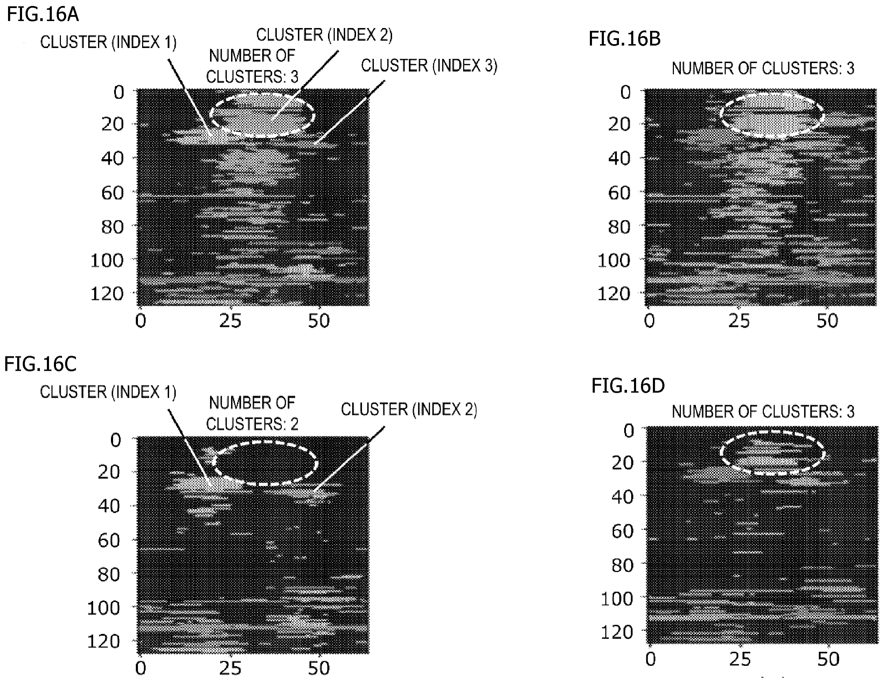

FIGS. 16A to 16D are diagrams illustrating results of clustering, and are maps of cluster indices classified by the clustering. The abscissa in FIGS. 16A to 16D indicates distance, and the ordinate in FIGS. 16A to 16D indicates azimuth. FIG. 16A illustrates a result of clustering in the period of the second slot illustrated in FIG. 7. FIG. 16B illustrates a result of clustering in the period of the third slot illustrated in FIG. 7. FIG. 16C illustrates a result of clustering in the period of the fourth slot illustrated in FIG. 7. FIG. 16D illustrates a result of clustering in the period of the fifth slot illustrated in FIG. 7. Note that since clustering indices are assigned in no particular order, different indices may be assigned to the same region for each slot as illustrated in FIGS. 16A and 16C. The periods of the second to fifth slots illustrated in FIG. 7 are static state periods, and it is determined that there is no body movement of the person within the measurement range. However, the number of clusters in the third slot has decreased in comparison with the number of clusters in the fourth slot. In addition, as illustrated in FIG. 16C, the respiratory signal in the region (bin) surrounded by the dotted line has not been detected. This means that in the period of the fourth slot, it is highly likely that there is a person whose breathing is disrupted due to causes such as apnea, shortness of breath, or coughing.

The abnormality detection unit 138 notifies the user of an occurrence of a vital abnormality of the person within the measurement range. The abnormality detection unit 138 or the output apparatus 114 may output a message indicating an occurrence of a vital abnormality of the person within the measurement range to the display apparatus 300. The abnormality detection unit 138 may notify an occurrence of a vital abnormality by turning on or blinking an indicator light provided in the processing apparatus 100, or by outputting voice or an alarm sound from the processing apparatus 100. In this manner, the user can determine that there is an occurrence of a vital abnormality of the person within the measurement range. The abnormality detection unit 138 notifies the user of the period in which the vital abnormality has occurred in the person within the measurement range (abnormal period). The abnormality detection unit 138 or the output apparatus 114 may output the data related to the abnormal period to the display apparatus 300. The abnormality detection unit 138 may notify the abnormal period by outputting voice from the processing apparatus 100. In this manner, the user can determine the abnormal period.

The present invention may be regarded as a processing system or a control system including at least some of the configurations, means, functions described above. The present invention may be regarded as a processing method or a control method including at least some of the processes described above. The present invention may be regarded as a method of executing the processes described above by a computer. The present invention may be regarded as a program for a computer to execute the processes described above, and the program may be provided to a computer through a network or from a computer-readable recording medium that non-transitorily stores data or the like.

### <Computer-Readable Recording Medium>

A program for causing a computer or other machine or apparatus (hereinafter referred to as "computer or the like") to implement any of the above-described functions may be recorded on a computer-readable recording medium. The functions can be provided by causing the computer or the like to read and execute the program from the recording medium.

Here, the term "computer-readable recording medium" refers to a recording medium that stores information such as data or programs by electrical, magnetic, optical, mechanical, or chemical means, and that can be read by a computer or the like. Examples of such recording media that are removable from the computer or the like include flexible disks, magneto-optical disks, CD-ROMs, CD-R/RWs, DVDs, Blu-ray Discs, and memory cards such as flash memory. Examples of recording media fixed to the computer or the like include hard disks and ROMs.

### <Supplementary Note 1>

A processing apparatus (100) including:
a signal acquisition unit (131) configured to acquire a vital signal from a signal reflected by at least one person within a measurement range;
an index computation unit (132) configured to compute a vital index representing a vital state of the at least one person based on the vital signal;
a body movement determination unit (133) configured to determine the presence or absence of a body movement of the at least one person within the measurement range;
a person count estimation unit (134) configured to estimate the number of the at least one person within the measurement range;
a person count change determination unit (135) configured to determine the presence or absence of a possibility of a change in the estimated number of the at least one person when the presence is determined of the body movement of the at least one person within the measurement range; and
a calculation unit (136) configured to calculate time-series data of the vital index for each of the at least one person, in which
when the presence is determined of the possibility of the change in the estimated number of the at least one person, the calculation unit (136) separately calculates the time-series data of the vital index before determining the presence of the possibility of the change in the estimated number of the at least one person, and the time-series data of the vital index after determining the presence of the possibility of the change in the estimated number of at least one person.

### <Supplementary Note 2>

The processing apparatus (100) according to Supplementary Note 1, in which the body movement determination unit (133) computes a body movement time of the at least one person within the measurement range, and
the person count change determination unit (135) determines the presence or absence of the possibility of the change in the estimated number of the at least one person based on the body movement time of the at least one person.

### <Supplementary Note 3>

The processing apparatus (100) according to Supplementary Note 1 or 2, in which
the index computation unit (132) computes the vital index for each of a plurality of periods,
the person count estimation unit (134) estimates the number of the at least one person within the measurement range for each of the plurality of periods,
the body movement determination unit (133) determines the presence or absence of the body movement of the at least one person within the measurement range for each of the plurality of periods, and
the calculation unit (136) calculates the time-series data of the vital index for each of the at least one person by excluding a period in which the vital index corresponding to the estimated number of the at least one person has not been computed from a period in which the absence is determined of the body movement of the at least one person within the measurement range among the plurality of periods.

### <Supplementary Note 4>

The processing apparatus (100) according to any one of Supplementary Notes 1 to 3, further including a location information computation unit (138) configured to identify a position of the person within the measurement range, and compute location information of the at least one person associated with the vital index for each of the at least one person, in which
the index computation unit (132) computes the vital index for each of a plurality of periods,
the location information computation unit (138) computes the location information for each of the plurality of periods, and
the calculation unit (136) determines a combination of the vital index for each of the at least one person by associating the vital index for each of the at least one person based on the location information for each of the plurality of periods, and calculates the time-series data of the vital index for each of the at least one person based on the combination of the vital index.

### <Supplementary Note 5>

The processing apparatus (100) according to any one of Supplementary Notes 1 to 3, in which
the index computation unit (132) computes the vital index for each of a plurality of periods, and
the calculation unit (136) determines a combination of the vital index for each of the at least one person by associating the vital index for each of the at least one person based on a value of the vital index for each of the plurality of periods,
and calculates the time-series data of the vital index for each of the at least one person based on the combination of the vital index.

### <Supplementary Note 6>

The processing apparatus (100) according to any one of Supplementary Notes 1 to 5, further including an index output unit (137) configured to output the time-series data of the vital index for each of the at least one person, in which when the presence is determined of the possibility of the change in the estimated number of the at least one person, the index output unit (137) separately outputs the time-series data of the vital index before determining the presence of the possibility of the change in the estimated number of the at least one person, and the time-series data of the vital index after determined the presence of the possibility of the change in the estimated number of at least one person.

### <Supplementary Note 7>

The processing apparatus (100) according to any one of Supplementary Notes 1 to 6, in which
the signal acquisition unit (131) executes a process of clustering a vital waveform group into a plurality of clusters based on a feature computed from the vital signal, the vital waveform group being acquired based on the vital signal, and
the person count estimation unit (134) estimates the number of the at least one person within the measurement range based on the number of clusters.

### <Supplementary Note 8>

The processing apparatus (100) according to Supplementary Note 7, in which the body movement determination unit (133) determines the presence or absence of the body movement of the at least one person within the measurement range for each of the plurality of periods, and
the person count estimation unit (134) estimates the number of the at least one person within the measurement range for each of the plurality of periods, and
estimates that a maximum value of the number of clusters is the number of the at least one person within the measurement range for a period in which the absence is determined of the body movement of the at least one person within the measurement range among a continuous period in the plurality of periods.

### <Supplementary Note 9>

The processing apparatus (100) according to Supplementary Note 7, further including an abnormality detection unit (137) configured to detect a vital abnormality of the at least one person within the measurement range based on the estimated number of the at least one person and the number of clusters.

### <Supplementary Note 10>

The processing apparatus (100) according to Supplementary Note 9, in which the body movement determination unit (133) determines the presence of absence of the body movement of the at least one person within the measurement range for each of the plurality of periods,
the person count estimation unit (134) estimates the number of the at least one person within the measurement range for each of the plurality of periods, and
estimates that a maximum value of the number of clusters is the number of the at least one person within the measurement range for a period in which the absence is determined of the body movement of the at least one person within the measurement range among a continuous period in the plurality of periods, and
the abnormality detection unit (137) determines that a period among the continuous period, in which the absence is determined of the body movement of the at least one person within the measurement range and the number of clusters is smaller than the maximum value is a period in which the vital abnormality of the at least one person has occurred.

### <Supplementary Note 11>

The processing apparatus (100) according to Supplementary Note 7, further including an abnormality detection unit (137) configured to detect a vital abnormality of the at least one person within the measurement range, in which the body movement determination unit (133) the presence or absence of the body movement of the at least one person within the measurement range for each of the plurality of periods,
the person count estimation unit (134) estimates the number of the at least one person within the measurement range for each of the plurality of periods, and estimates that a maximum value of the number of clusters is the number of the at least one person within the measurement range for a period in which the absence is determined of the body movement of the at least one person within the measurement range among a continuous period in the plurality of periods, and
the abnormality detection unit (137) determines whether a vital abnormality has occurred in the at least one person within the measurement range based on the vital signal in a period immediately preceding a period among the continuous period in which the absence is determined of the body movement of the at least one person within the measurement range and the number of clusters is smaller than the maximum value.

### <Supplementary Note 12>

A processing method configured to be executed by a processing apparatus (100), the method including:
acquiring a vital signal from a signal reflected by at least one person within a measurement range;
computing a vital index representing a vital state of the at least one person based on the vital signal;
determining the presence of absence of a body movement of the at least one person within the measurement range;
estimating the number of the at least one person within the measurement range;
determining the presence or absence of a possibility of a change in the estimated number of the at least one person when the presence is determined of the body movement of the person within the measurement range; and
calculating time-series data of the vital index for each of the at least one person, wherein
when the presence is determined of the possibility of the change in the estimated number of the at least one person, the calculating includes separately calculating the time-series data of the vital index before determining the presence of the possibility of the change in the estimated number of the at least one person, and the time-series data of the vital index after determining the presence of the possibility of the change in the estimated number of the at least one person.

### <Supplementary Note 13>

A program configured to cause a computer to execute:
acquiring a vital signal from a signal reflected by at least one person within a measurement range;
computing a vital index representing a vital state of the at least one person based on the vital signal;
determining the presence of absence of a body movement of the at least one person within the measurement range;
estimating the number of the at least one person within the measurement range;
determining the presence or absence of a possibility of a change in the estimated number of the at least one person when the presence is determined of the body movement of the at least one person within the measurement range; and
calculating time-series data of the vital index for each of the at least one person, in which
when the presence is determined of the possibility of the change in the estimated number of the at least one person, the calculating includes separately calculating the time-series data of the vital index before determining the presence of the possibility of the change in the estimated number of the at least one person, and the time-series data of the vital index after determining the presence of the possibility of the change in the estimated number of the at least one person.

### REFERENCE SIGNS

100: Processing apparatus
111: Transmission/reception apparatus
112: Control apparatus
113: Storage apparatus
114: Output apparatus
121: Transmission unit
122: Reception unit
131: Signal acquisition unit
132: Index computation unit
133: Body movement determination unit
134: Person count estimation unit
135: Person count change determination unit
136: Calculation unit
137: Index output unit
138: Abnormality detection unit
139: Location information computation unit
151: Signal processing unit
152: Clustering unit

## Claims

1. A processing apparatus comprising:
a signal acquisition unit configured to acquire a vital signal from a signal reflected by at least one person within a measurement range;
an index computation unit configured to compute a vital index representing a vital state of the at least one person based on the vital signal;
a body movement determination unit configured to determine the presence or absence of a body movement of the at least one person within the measurement range;
a person count estimation unit configured to estimate the number of the at least one person within the measurement range;
a person count change determination unit configured to determine the presence or absence of a possibility of a change in the estimated number of the at least one person when the presence is determined of the body movement of the person within the measurement range; and
a calculation unit configured to calculate time-series data of the vital index for each of the at least one person, wherein
when the presence is determined of the possibility of the change in the estimated number of the at least one person, the calculation unit separately calculates the time-series data of the vital index before determining the presence of the possibility of the change in the estimated number of the at least one person, and the time-series data of the vital index after determining the presence of the possibility of the change in the estimated number of the at least one person.

2. The processing apparatus according to claim 1, wherein
the body movement determination unit computes a body movement time of the at least one person within the measurement range, and
the person count change determination unit determines the presence or absence of the possibility of the change in the estimated number of the at least one person based on the body movement time of the at least one person.

3. The processing apparatus according to claim 1, wherein the index computation unit computes the vital index for each of a plurality of periods,
the person count estimation unit estimates the number of the at least one person within the measurement range for each of the plurality of periods, the body movement determination unit determines the presence or absence of the body movement of the at least one person within the measurement range for each of the plurality of periods, and
the calculation unit calculates the time-series data of the vital index for each of the at least one person by excluding a period in which the vital index corresponding to the estimated number of the at least one person has not been computed from a period in which the absence is determined of the body movement of the at least one person within the measurement range among the plurality of periods.

4. The processing apparatus according to claim 1, further comprising a location information computation unit configured to identify a position of the at least one person within the measurement range, and compute location information of the at least one person associated with the vital index for each of the at least one person, wherein
the index computation unit computes the vital index for each of a plurality of periods,
the location information computation unit computes the location information for each of the plurality of periods, and
the calculation unit determines a combination of the vital index for each of the at least one person by associating the vital index for each of the at least one person based on the location information for each of the plurality of periods, and calculates the time-series data of the vital index for each of the at least one person based on the combination of the vital index.

5. The processing apparatus according to claim 1, wherein
the index computation unit computes the vital index for each of a plurality of periods, and
the calculation unit determines a combination of the vital index for each of the at least one person by associating the vital index for each of the at least one person based on a value of the vital index for each of the plurality of periods, and calculates the time-series data of the vital index for each of the at least one person based on the combination of the vital index.

6. The processing apparatus according to claim 1, further comprising an index output unit configured to output the time-series data of the vital index for each of the at least one person, wherein
when the presence is determined of the possibility of the change in the estimated number of the at least one person, the index output unit separately outputs the time-series data of the vital index before determining the presence of the possibility of the change in the estimated number of the at least one person, and the time-series data of the vital index after determining the presence of the possibility of the change in the estimated number of the at least one person.

7. The processing apparatus according to any one of claims 1 to 6, wherein
the signal acquisition unit executes a process of clustering a vital waveform group into a plurality of clusters based on a feature computed from the vital signal, the vital waveform group being acquired based on the vital signal, and
the person count estimation unit estimates the number of the at least one person within the measurement range based on the number of clusters.

8. The processing apparatus according to claim 7, wherein
the body movement determination unit determines the presence or absence of the body movement of the at least one person within the measurement range for each of the plurality of periods, and
the person count estimation unit estimates the number of the at least one person within the measurement range for each of the plurality of periods, and estimates that a maximum value of the number of clusters is the number of the at least one person within the measurement range for a period in which the absence is determined of the body movement of the at least one person within the measurement range among a continuous period in the plurality of periods.

9. The processing apparatus according to claim 7, further comprising an abnormality detection unit configured to detect a vital abnormality of the at least one person within the measurement range based on the estimated number of the at least one person and the number of clusters.

10. The processing apparatus according to claim 9, wherein
the body movement determination unit determines the presence of absence of the body movement of the at least one person within the measurement range for each of the plurality of periods,
the person count estimation unit estimates the number of the at least one person within the measurement range for each of the plurality of periods, and estimates that a maximum value of the number of clusters is the number of the at least one person within the measurement range for a period in which the absence is determined of the body movement of the at least one person within the measurement range among a continuous period in the plurality of periods, and
the abnormality detection unit determines that a period among the continuous period, in which the absence is determined of the body movement of the at least one person within the measurement range and the number of clusters is smaller than the maximum value is a period in which the vital abnormality of the at least one person has occurred.

11. The processing apparatus according to claim 7, further comprising an abnormality detection unit configured to detect a vital abnormality of the at least one person within the measurement range, wherein
the body movement determination unit determines the presence or absence of the body movement of the at least one person within the measurement range for each of the plurality of periods,
the person count estimation unit estimates the number of the at least one person within the measurement range for each of the plurality of periods, and estimates that a maximum value of the number of clusters is the number of the at least one person within the measurement range for a period in which the absence is determined of the body movement of the at least one person within the measurement range among a continuous period in the plurality of periods, and
the abnormality detection unit determines whether a vital abnormality has occurred in the at least one person within the measurement range based on the vital signal in a period immediately preceding a period among the continuous period in which the absence is determined of the body movement of the at least one person within the measurement range and the number of clusters is smaller than the maximum value.

12. A processing method executed by a processing apparatus, the processing method comprising:
acquiring a vital signal from a signal reflected by at least one person within a measurement range;
computing a vital index representing a vital state of the at least one person based on the vital signal;
determining the presence of absence of a body movement of the at least one person within the measurement range;
estimating the number of the at least one person within the measurement range;
determining the presence or absence of a possibility of a change in the estimated number of the at least one person when the presence is determined of the body movement of the person within the measurement range; and
calculating time-series data of the vital index for each of the at least one person, wherein
when the presence is determined of the possibility of the change in the estimated number of the at least one person, the calculating includes separately calculating the time-series data of the vital index before determining the presence of the possibility of the change in the estimated number of the at least one person, and the time-series data of the vital index after determining the presence of the possibility of the change in the estimated number of the at least one person.

13. A program for causing a computer to execute:
acquiring a vital signal from a signal reflected by at least one person within a measurement range;
computing a vital index representing a vital state of the at least one person based on the vital signal;
determining the presence of absence of a body movement of the at least one person within the measurement range;
estimating the number of the at least one person within the measurement range;
determining the presence or absence of a possibility of a change in the estimated number of the at least one person when the presence is determined of the body movement of the at least one person within the measurement range; and
calculating time-series data of the vital index for each of the at least one person, wherein
when the presence is determined of the possibility of the change in the estimated number of the at least one person, the calculating includes separately calculating the time-series data of the vital index before determining the presence of the possibility of the change in the estimated number of the at least one person, and the time-series data of the vital index after determining the presence of the possibility of the change in the estimated number of the at least one person.
